Europäisches Patentamt

European Patent Office

Office européen des brevets

(19)

(11) Veröffentlichungsnummer: **0 094 560**
**B1**

(12) ## EUROPÄISCHE PATENTSCHRIFT

(45) Veröffentlichungstag der Patentschrift:
24.09.86

(51) Int. Cl.⁴: **C 07 C 135/02**

(21) Anmeldenummer: 83104407.8

(22) Anmeldetag: 05.05.83

(54) Verfahren zur Herstellung von 3-(10,11-Dihydro-5H-dibenzo /a,d/-cyclohepten-5-yliden)-N,N-dimethyl-1-propanamin-N-oxid-Dihydrat.

(30) Priorität: 13.05.82 DE 3218026

(43) Veröffentlichungstag der Anmeldung:
23.11.83 Patentblatt 83/47

(45) Bekanntmachung des Hinweises auf die Patenterteilung:
24.09.86 Patentblatt 86/39

(84) Benannte Vertragsstaaten:
AT BE CH DE FR GB IT LI LU NL SE

(56) Entgegenhaltungen:
DE-A-1 543 224
DE-A-2 557 456
DE-B-1 221 645
DE-B-1 243 180
DE-B-1 518 104
US-A-3 432 555

THE MERCK INDEX, 9th edition, 1976 Merck & Co.,
INC. RAHWAY, N.J., U.S.A. Seite ONR-19
JOURNAL OF PHARMACEUTICAL SCIENCES Vol.
64, No. 9, September 1975 R.P. ENEVER et al.
"Decomposition of Amitriptyline Hydrochloride in
Aqueous Solution: Identification of Decomposition
Products" Seiten 1497-1499

(73) Patentinhaber: A. Nattermann & Cie. GmbH,
Nattermannallee 1, D-5000 Köln 30 (DE)

(72) Erfinder: Günther, Bernd-Rainer, Dr., Auf der
Höhe 31, D-5010 Bergheim (DE)

(74) Vertreter: Redies, Bernd, Dr. rer. nat., COHAUSZ
& FLORACK Patentanwaltsbüro
Schumannstrasse 97 Postfach 14 01 47, D-4000
Düsseldorf 1 (DE)

LIBER, STOCKHOLM 1986

## Beschreibung

Die vorliegende Erfindung betrifft ein neues Verfahren zur Herstellung von 3-(10,11-Dihydro-SH-dibenzo [a, d]-cyclohepten-5-yliden)-N,N-dimethyl-1-propanamin-N-oxid-Dihydrat und deren Säureadditionssalze gemäß Anspruch 1 und 2.

3-(10,11-Dihydro-S-dibenzo [a, d]-cyclohepten-S-yliden)-N,N-dimethyl-1-propanamin-N-oxid-Dihydrat (Amitriptylinoxid) hat sich bei der Behandlung von Depressionen bewährt (N. Nadjafi, Med. Welt 1981, 32 (40), 1497-1500). Das N-Oxid läßt sich, wie in der DE-PS 12 43 180 beschrieben, aus 3-(10,11-Dihydro-5H-dibenzo [a, d]-cyclohepten-5-yliden)-N,N-dimethyl-1-propanamin in methanolischer Lösung durch Umsetzung mit 30%igem Wasserstoffperoxid herstellen. Nach einer mindestens 7tägigen Reaktionszeit wird das Reaktionsgemisch mit Wasser verdünnt, das Methanol im Vakuum abdestilliert und das N-Oxid bzw. das entstehende N-Oxid-Dihydrat aus der wässrigen Phase isoliert. Dieses Verfahren ist sehr zeitaufwendig und ist aus sicherheitstechnischer Sicht durch die Verwendung eines organischen Lösungsmittels unbefriedigend.

In einem anderen Verfahren, DE-OS 15 43 224. wird 3-(10,11-Dihydro-5H-dibenzo [a, d]-cyclohepten-5-yliden)-N,N-dimethyl-1-propanamin in methanolischer Lösung mit einem Oberschuß eines 30%igen Wasserstoffperoxids 30 Min. in der Kälte und anschließend 24 Stunden bei Raumtemperatur umgesetzt. Das überschüssige Peroxid muß mit Platin reduziert werden, muß weitere Stunden gerührt und dann 24 Stunden stehen gelassen werden. Danach wird das Platin durch Filtration entfernt. Aus dem Filtrat kann dann das N-Oxid isoliert werden.

Auch bei diesem Verfahren muß in organischen Lösungsmitteln gearbeitet werden. Weiterhin ist auch dieses Verfahren sehr zeitaufwendig, insbesondere durch zusätzliche Verfahrensschritte, wie z.B. die Reduzierung von überschüssigem Peroxid durch Platin.

Wird bei der Reaktion ohne Lösungsmittel bei Raumtemperatur gearbeitet, so wird nach ca. 15 Stunden ein Umsatz von ca. 50 % erzielt, wobei das entstandene N-Üxid ausfällt und das Reaktionsgemisch dadurch nicht mehr gerührt werden kann. Damit ist die weitere Umsetzung erschwert. Bei einer Erhöhung der Reaktionstemperatur mußte wegender thermischen Instabilität des N-Oxids mit einer Verringerung der Ausbeute gerechnet werden.

Ziel der vorliegenden Erfindung ist es deshalb, ein einfaches Verfahren zur Herstellung von 3-(10,11-Dihydro-SH-dibenzo [a, d]-cyclohepten-S-yliden)-N,N-dimethyl-1-propan-amin-N-oxid-Dihydrat und dessen Säureadditionssalze zur Verfügung zu stellen, welches ohne organische Lösungsmittel in kurzer Reaktionszeit hohe Ausbeute liefert.

Es wurde nun Überraschend gefunden, daß man 3-(10,11-Di-hydro-5H-dimethyl-1-propanamin-N-oxid-Dihydrat aus 3-(10,11-Dihydro-5H-dibenzo[a, d] -cyclohepten-5-yliden)-N,N-dimethyl-1-propanamin bei 30 - 95°C, insbes. bei 50 - 6S°C durch Umsetzung mit 25 - 85%igem, insbes. mit 25 - 35%igem Wasserstoffperoxid nach Reaktionszeiten von 20 Minuten bis 4 Stunden, insbes. 1 - 2-Stunden, in hochreiner Form in höher Ausbeute gewinnen kann. Aus der Base kann man die entsprechenden Säureadditionssalze durch Umsetzung der Base mit der entsprechenden Säure in bekannter Weise umsetzen.

Dies muß als überraschend angesehen werden, da zu erwarten war, daß bei höheren Reaktionstemperaturen das N-Oxid sich über die Cope Eliminations Reaktion (Merck Index - Nith Ed. 1976 - ONR -19) zum wesentlich stabilieren 5-Allyliden-10 11-dihydro-5H-dibenzo[a,d]cyclohepta[1,4]dien umlagert (R.P.Enever et al., J. Pharm. Sci 1975, 64(9), 1497-1499).

### Beispiel 1

60g (0,22 Mol) 3-(10,11-Dihydro-5H-dibenzo44Aa,d45A-cyclohepten-5-yliden)-N,N-dimethyl-1-propanamin werden auf 50°C erwärmt. Innerhalb von 30 Minuten werden 26 g 30%ige Wasserstoffperoxidlösung bei 50 - 65°C zugetropft. Man läßt eine Stunde bei 55 - 65°C nachrühren und fügt dann 120 ml Wasser zu. Dabei kristallisiert 3-(10,11-Dihydro-5H-dibenzo [a,d]-cyclohepten-5-yliden)-N,N-dimethyl-1-propanamin-N-oxid-Dihydrat aus. Das Gemisch wird eine Stunde bei Raumtemperatur gerührt, das N-Oxid abgesaugt, mit Wasser gewaschen und im Vakuum bei Raumtemperatur getrocknet.

Ausbeute: 66,3g (93% d. Th.) 3-(10,11-Dihydro-5H-dibenzo [a,d]-cyclohepten-5-yliden)-N,N-dimethyl-1-propanamin-N-oxid-Dihydret mit einem 5chmelzpunkt von 102-103°C.

### Beispiel 2

60g (0,22 Mol) 3-(10,11-Dihydro-5H-dibenzo [-a,d]-cyclohepten-5-yliden)-N,N-dimethyl-1-propanamin werden auf 80°C erwärmt. Innerhalb von 30 Minuten werden 26g 30%iger Wasserstoffperoxidlösung bei 85-95°C zugetropft. Man läßt 20 Minuten nachrühren und arbeitet entsprechend Beispiel 1 auf. Ausbeute: 50,5g (70% d. Th.) 3-(10,11-Dihydro-5H-dibenzo [a,d]-cyclohepten-5-yliden)-N,N-dimethyl-1-propanamin-Noxid-Dihydrat.

### Beispiel 3

60g (0,22 Mol) 3-(10,11-Dihydro-5H-dibenzo [a,d]-cyclohepten-5-yliden)-N,N-dimethyl-1-

propanamin werden auf 30-35°C erwärmt. Innerhalb von 2 Stunden werden 26g 30%iger Wasserstoffperoxidlösung bei 30-35°C zugetropft. Man läßt 4 Stunden nachrühren und arbeitet entsprechend Beispiel 1 auf. Ausbeute: 36g (50% d. Th.) 3-(10,11-Dihydro-5H-dibenzo [a,d] -cyclohepten-5-yliden)-N,N-dimethyl-1-propanamin-Noxid-Dihydrat.

**Patentansprüche**

1. Verfahren zur Herstellung von 3-(10,11-Dihydro-5H-dibenzo [a,d] -cyclohepten-5-yliden)-N,N-dimethyl-1-propanamin-N-oxid-Dihydrat und dessen Säureadditionssalzen, dadurch gekennzeichnet, daß man 3-(10,11-Dihydro-5H-dibenzo [a,d] -cyclohepten-5-yliden)-N,N-dimethyl-1-propanamin mit 25 - 85%iger Wasserstoffperoxidlösung bei 30 - 95°C 20 Minuten bis 4 Stunden zur Reaktion bringt, worauf das erhaltene N-Oxid als solches oder als Säure-Additionssalz aus der Reaktionsmischung in an sich bekannter Weise isoliert wird.

2. Verfahren zur Herstellung von 3-(10,11-Dihydro-5H-dibenzo [a,d] -cyclohepten-5-yliden)-N,N-dimethyl-1-propanamin-N-oxid-Dihydrat und dessen Säureadditionssalzen gemäß Anspruch 1, dadurch gekennzeichnet, daß die Reaktion mit 25 - 35%igem Wasserstoffperoxid bei 50-85°C in 1-2 Stunden durchgeführt wird.

**Claims**

1. Process for the preparation of 3-(10,11-dihydro-5H-dibenzo [a,d]-cyclohepten-5-ylidene)-N,N-dimethyl-1-propanamine-N-oxide dihydrate and its acid addition salts which comprises reacting 3-(10,11-dihydro-5H-dibenzo [a,d]-cyclohepten-5-ylidene)-N,N-dimethyl-1-propanamine with 25 - 85 % hydrogen peroxide solution at 30 - 95°C for a period of 20 minutes to 4 hours, isolating the resulting N-oxide from the reaction mixture as such or as acid addition salt in known manner.

2. Process for preparation of 3-(10,11-dihydro-5H-dibenzo [a,d]-cyclophepten-5-ylidene)-N,N-dimethyl-1-propanamine-N-oxide dihydrate and its acid addition salts as claimed in claim 1, wherein the hydrogen peroxide solution has a strength in the range 25 - 35 % at a reaction temperature in the range 50 - 65°C for a reaction period of 1 to 2 hours.

**Revendications**

1. Procédé de préparation de 3-(10,11-dihydro-5H-dibenzo[a,d]-cycloheptène-5-ylidène)-N,N-dimétyl-1-propanamine-N-oxyde dihydraté et de ses sels d'addition d'acides, caractérisé en ce qu'on fait réagir la 3-10,11-dihydro-5H-dibenzo[a,d]-cycloheptène-5-ylidène)-N,N-diméthyl-1-propanamine avec une solution de peroxyde d'hydrogène à 25-85% à 30-95°C pendant 20 minutes à 4 heures, après quoi on isole de façon connue à partir du mélange réactionelle le N-oxyde obtenue tel quel ou sous forme de sel d'addition d'acide.

2. Procédé de préparation de 3-(10,11-dihydro-5H-dibenzo[a,d]-cycloheptène-5-ylidène)-N,N-deméthyl-1-propanamine-N-oxyde dihadraté et de ses sels d'addition d'acides selon la revendication 1, caractérisé en ce qu'on conduit la réaction avec du peroxyd d'hydrogène à 25-35% à 50-65°C en 1-2 heures.